# EUROPEAN PATENT SPECIFICATION

(11) **EP 2 020 999 B1**
(45) Date of publication and mention of the grant of the patent: **31.08.2011**
(21) Application number: 07728341.4
(22) Date of filing: 20.04.2007
(51) Int. Cl.: A61K 31/198, A61P 21/00

(54) **COMPOSITIONS COMPRISING ALPHA-KETOGLUTARATE AND THEIR USE FOR MODULATING MUSCLE PERFORMANCE**
ZUSAMMENSETZUNGEN UMFASSEND ALPHA-KETOGLUTARAT UND IHRE VERWENDUNG ZUR MODULIERUNG DER MUSKELLEISTUNG
COMPOSITIONS RENFERMANT DE L'ALPHA-CETOGLUTARATE ET LEUR UTILISATION POUR MODULER LA PERFORMANCE MUSCULAIRE

(30) Priority: 21.04.2006 PL 37951206
(43) Date of publication of application: 11.02.2009
(73) Proprietor: SGP & SONS AB, 211 15 Malmö (SE)
(72) Inventor: PIERZYNOWSKI, Stefan, 211 14 Malmö (SE)
(74) Representative: Bengtsson, Peggy Katrin
(86) International application number: PCT/EP2007/053882
(87) International publication number: WO 2007/122190

(56) References cited:
- EP-A1- 0 318 446
- WO-A-95/34301
- WO-A-03/010131
- WO-A-2005/002567
- US-A- 5 817 364
- WALSER M; MITCH W E; ABRAS E: "SUPPLEMENTS CONTAINING AMINO-ACIDS AND KETO ACIDS IN THE TREATMENT OF CHRONIC UREMIA" KIDNEY INTERNATIONAL SUPPLEMENT, no. 16, 1983, pages S285-S289, XP009088592 3RD INTERNATIONAL CONGRESS ON NUTRITION AND METABOLISM IN RENAL DISEASE, MARSEILLES, FRANCE, SEPT. 1 ISSN: 0098-6577

## Description

### TECHNICAL FIELD

This invention relates to a medicament for use in modulating muscle performance in a mammal including a human being. Also contemplated is a medicament for use in modulating muscle performance in a vertebrate, including mammal and bird, and the manufacture of a medicament for the prevention, alleviation or treatment of muscle performance in said vertebrate.

### BACKGROUND OF THE INVENTION

*Muscle performance in uremic patients.* Patients with end-stage renal disease (uremia) have limited physical fitness, which is often the cause of subsequent problems, namely cardiac dysfunction, muscle abnormalities and even depression (Gutman et al. 1981; Painter, 1988, Kouidi et al. 1997; Shalom et al. 1984). Yet, whilst muscle weakness is a common complaint amongst dialysis patients it remains an unexplained phenomenon, and therefore not easily treated, which severly affects their daily life whether it be work- or recreation related (Nakao et al. 1982).

In recent years research into renal failure and uremic myopathy has focused on the training aspect of patient health. Indeed, it has been shown that exercise training can significantly reduce the muscle atrophy that occurs in patients with end-stage renal disease on haemodialysis (Shalom et al. 1984; Painter, 1988; Eidemak et al. 1997; Kouidi et al. 1998). To date, however, there is a limited knowledge of the changes that occur in muscle function and structure with the progression of this disease, or indeed the restorative effects that exercise training can have.

It is known that muscle weakness, a symptom of uremia, is associated with an unequivocal loss of muscle force (typically a loss of 140 Newtons in *quadriceps* muscles of patients vs. control subjects; Fahal et al. 1997). It is believed, though, that the problem is not one of impaired excitation-contraction coupling - as this would be associated with a selective loss of force at low excitation frequencies which has not been observed.

Muscle atrophy or loss is fibre specific, in that uremia is associated mostly with a loss of type II fibres and predominantly the type IIb fibres which are fast-twitch and glycolytic in nature (Fahal et al. 1997), although atrophy of type I (slow-twitch, oxidative fibers) also occurs in some patients (Moore et al. 1993).

*Muscle strength determined bone strength.* Two new studies show that people with strong muscles also have stronger bones. A study from Johns Hopkins shows that heart and lung fitness is not associated with stronger bones, but that muscle strength is (Stewart et al. 2002).

Furthermore, a second study from Turkey shows that grip pressure is closely and positively associated with bone strength (Sahin et al. 2002).

Thus, it is the object of the invention to develop means for treating or preventing any condition associated with decreased muscle performance, such as uremia, and which, thus, increases muscle performance, which can also avoid problems associated with prior art means. In this respect, the present invention addresses this need and interest.

### SUMMARY OF THE INVENTION

The present invention also provides a medicament for use in modulating muscle performance in a mammal, comprising alpha-ketoglutarate (AKG) or mono- and bivalent metal salts of alpha-ketoglutarate or ornithine alpha-ketoglutarate and, additionally, optional glucose.

Preferably, the medicament for use in modulating muscle performance in a mammal, is for administration orally in a dose of 5-40 mg/kg bodyweight or parenterally in a dose of 0.1-0.4 mg/kg bodyweight and the medicament comprising optionally glucose in an amount of 0.1-5 mM. Preferably, the medicament is for administration orally or parenterally to the vertebrate selected from the group consisting of a rodent, such as a mouse, rat, guinea pig, or a rabbit; farm animals, such as a cow, horse, pig, piglet; a bird, such as a hen and turkey; free going farm animals or birds; and a pet, such as a dog, or a cat, being in the need thereof, as well as a human being.

Furthermore, a medicament for use in the prevention, alleviation or inhibition of muscle performance in a vertebrate is provided, comprising alpha-ketoglutarate (AKG) or mono- and bivalent metal salts of alpha-ketoglutarate or ornithine alpha-ketoglutarate and, additionally, optional glucose.

Preferably, in the medicament for use in the prevention, alleviation or inhibition of muscle performance in a vertebrate, is for administration orally in a dose of 5-40 mg/kg bodyweight or parenterally in a dose of 0.1-0.4 mg/kg bodyweight or via dialysis in a dose 1-1000 mg/kg bodyweight, the medicament comprising optionally glucose in an amount of 0.1-5 mM. Preferably, the medicament is administered orally or parenterally, especially via dialysis, to the vertebrate selected from the group consisting of a rodent, such as a mouse, rat, guinea pig, or a rabbit; farm animals, such as a cow, horse, pig, piglet; a bird, such as a hen and turkey; free going farm animals or birds; and a pet, such as a dog, or a cat, being in the need thereof, as well as a human being.

The present invention relates also to the use of alpha-ketoglutarate (AKG), mono- or divalent metal salts of AKG or ornithine-AKG for manufacturing a medicament for the prevention, alleviation or inhibition of muscle performance in a vertebrate and for manufacturing means for modulating muscle performance in a mammal.

Preferably, the medicament is a pharmaceutical composition with pharmaceutically acceptable carriers or/and additives and is used for oral or parenteral administration.

The pharmaceutical composition is manufactured in the therapeutically effective amount of 5-40 mg/kg bodyweight per daily dose for an oral composition or 0.1-0.4 mg/kg bodyweight per daily dose for a parenteral composition or 1-1000 mg/kg bodyweight per daily dose for a dialysis composition, wherein the optional glucose is in amount of 0.1-5 mM.

### TECHNICAL BENEFITS OF THE INVENTION

The present invention provides a medicament comprising AKG composition and optionally glucose for use in improving muscle performance in case of a decreased muscle performance compared to what is normal for a subject.

The subject may be a vertebrate, such as a mammal or a bird, and examples are given in detail below. The vertebrate may, thus, be in the need thereof to increase muscle performance due to medical reasons.

The method comprises the step of administering a composition comprising alpha-ketoglutarate and glucose to said vertebrate in a sufficient amount and/or at a sufficient rate for a desired effect. The muscle performance is considered normal in a vertebrate, such as a mammal or a bird, not having a decrease in muscle performance and not taking said composition. The muscle performance is further considered increased when a higher muscle performance in observed or measured, as compared to a mammal of the same species not taking said composition. The increase in muscle performance may also be an increase as compared to the initial starting values of muscle performance in a vertebrate before administration of said composition.

The present invention also provides a medicament for use in modulating muscle performance in a mammal, comprising AKG and optionally glucose, for modulating muscle performance. The invention further provides the use of AKG and, optionally, glucose for the manufacture of a medicament for the prevention, alleviation or treatment of a decrease in muscle performance.

Still furthermore, the invention provides the use of AKG and, optionally, glucose for the manufacture of a medicament for the modulation of muscle performance in a vertebrate, including mammal, such as a human being, and bird.

### SHORT DESCRIPTION OF DRAWINGS

Fig. 1 presents a diagram showing the decrease in isometric force of soleus muscles from 4-wk-old rats during continuous electrical stimulation at 90 Hz.
Fig. 2 presents contracting characteristics of soleus muscles in Uremic Buffer (n=8) or Krebs Ringer physiological solution (n=9) during continuous electrical stimulation at 40 Hz (32 mA, 1 ms, <90V).
Fig. 3 presents contracting characteristics of extensor digitorum longus muscle in Uremic Buffer (n=7) or Krebs Ringer physiological solution (n=8) during continuous electrical stimulation at 90 Hz (32 mA, 1 ms, <90V).
Fig. 4 presents contracting characteristics of soleus muscles in uremic rats + test component (n=10), control rats + placebo (n=8), control rats + test component (n=4) incubated in Krebs Ringer physiological solution (30 min. incubation) and electrically stimulated at 40 Hz (32 mA, 1 ms, <90V).
Fig. 5 presents contracting characteristics of extensor digitorum longus muscle in uremic rats + test component (n=3) and control rats + test component (n=2) incubated in Krebs Ringer solution and uremic rats + test component (n=3) incubated in Uremic Buffer (30 min.), 90 Hz, 1 ms and <90V.

### DETAILED DESCRIPTION OF THE INVENTION

### Definitions

The term "muscle endurance" is herein intended to mean the ability of muscle groups to perform submaximal contractions for extended periods of time as defined by Zachazewski (1996, cited in Wheeler, T. Exercise Physiology 552 (1997) Review). The exact enumeration of parameters is difficult to define precisely since much depends on the amount of muscle force being generated, and the individual assessment of the point of exhaustion - thus, one could imagine that an individual performing at a very low level of muscle activity could continue for some hours without becoming tired, whilst another individual performing at near maximal muscle activity could only continue for a short period of time. Muscle endurance measurements take little account of the force being produced and focus more on the time aspect of muscular activity.

The term "muscle strength" is herein intended to mean either absolute strength and relative strength, independent of sex so long as muscles of identical size are studied. Thus, in absolute terms, the greater the cross sectional area of a muscle, the stronger the muscle, independent of sex (Martin, L. Exercise Physiology 552 (1997) Review). However, since men tend to have a greater upper body muscle mass, with longer muscles of a greater cross sectional area, men tend to be stronger than women. When strength is considered relative to body weight or lean body mass it is termed relative muscle strength. Most commonly strength is divided by body mass, fat free body mass, muscle cross sectional area, limb volume, girth, and height. By dividing strength by these factors the differences between males and females are significantly reduced or eliminated. Researchers have studied female performances and show that when performances are expressed per unit of lean body weight then female performances equal or exceed those of their male counterparts. It is for this reason that female swimming performances are closer to those of their male counterparts than for any other sport.

The term "muscle force" is herein intended to mean a component of the work performed by a muscle, calculated as WORK = force x distance expressed in SI units (N/g wet wt.), where distance is the measurement between the fulcrum and the point of force being exerted. Force can be readily measured using a transducer and expressed in terms of time to give an index of contractions over a period of sustained muscle activity. Close correlations exist between the force curve of an active muscle and the RMS of an electromyography recording. Thus, none invasive measurements of muscle activity can be obtained through EMG recordings and interpreted in terms of muscle force through the RMS transformation.

The term "modulating muscle performance" is herein intended to mean changing, modifying or otherwise influencing the muscle performance in a subject.

The term "improved muscle performance" is herein intended to mean changes in muscle performance, where the changes are compared to a subject of the same species not obtaining treatment or administration according to the invention. The changes are regarded as an improvement if such changes are positive for said mammal. Normally, an improved muscle performance is an increased muscle performance.

The term "parenteral" is herein intended to mean administration not through the alimentary canal but rather by injection through some other As used herein, "pharmaceutical composition" means therapeutically effective composition according to the invention.

A "therapeutically effective amount", or "effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce, alleviate and/or prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent; i.e., carrier, or additive.

As used herein, "treating", means treating for curing which may be a full curing or a partial curing of a decreased muscle performance.

As used herein "alleviation", means a decreased, i.e. less, or milder condition or conditions associated with a decreased muscle performance.

As used herein "preventing", means a complete or partial block of development, or outbreak, of a decreased muscle performance.

The term "derivate" or "derivative" is herein intended to mean a chemical substance derived from a mother substance either directly or by modification or partial substitution.

The term "analogue" or analog" is herein intended to mean compounds that are structurally similar to another, but are not necessarily isomers. Analogs have similar function(s) but differ in structure or evolutionary origin.

In the use for manufacture of medicaments of the invention, a therapeutically effective amount of the active component is provided. A therapeutically effective amount can be determined by the ordinary skilled medical or veterinary worker based on patient characteristics, such as age, weight, sex, condition, complications, other diseases, etc., as is well known in the art.

*A medicament for use in modulating muscle performance.* According to the invention, a medicament for use in modulating muscle performance in a vertebrate is included. The medicament comprises administering to said vertebrate in a sufficient amount and/or at a sufficient rate to enable a desired effect, AKG and further, optionally, glucose.

The modulation may be an increase in muscle performance compared to before treatment of that particular subject or compared to a subject not obtaining the treatment. Any modulation may change the muscle performance from a decreased level in direction of a normal or even higher level. The modulation may also be from a normal muscle performance level to a level above normal muscle performance of that particular species. What is considered as a normal muscle performance of that particular species is dependent of the species per se, age and sex. Any modulation leading to a beneficial change in muscle performance, i.e. a change towards a normal muscle performance or above normal muscle performance is considered as an improvement.

In still a further embodiment, the modulation may be an inhibition, prevention or alleviation of a decreased muscle performance. An inhibition may be a complete or partial inhibition in a mammal in the need thereof, such as a human being. A prevention may be a prophylactic treatment of a mammal in the need thereof, such as a human being.

Furthermore, a medicament for use in the inhibition, prevention or alleviation of a decrease in muscle performance in a vertebrate is included. The medicament comprises AKG and further optionally glucose.

In further embodiments, the vertebrate is selected from the group consisting of a rodent, such as a mouse, rat, guinea pig, or rabbit; farm animals, such as a cow, horse, pig, piglet; a bird, such as a hen and turkey; free going farm animals or birds; and a pet, such as a dog or cat.

In still an even further embodiment, the mammal is a human being. The human being may be a patient in the need of treatment of decreased muscle performance, an athlete, a malnutritioned human being, or an athlete, body builder, an astronaut, or a human being in the need thereof due to any condition.

Administration of AKG and, optionally, glucose, in the disclosed medicament, may be performed in different ways depending on what species of vertebrate to treat, the condition of the vertebrate in the need of said medicaments, and the specific indication to treat.

*Administration formats.* The dosage forms may include capsules or tablets, such as chewable or soluble, e.g. effervescent tablets, as well as powder and other dry formats known to the skilled man in the art, such as pellets, such as micropellets, and granules.

The intended administration may be parenteral, rectal or oral as e.g. a food or feed supplement. Parenteral vehicles include sodium chloride solution, Ringer's dextrose, dextrose and sodium chloride, lactated Ringer's or fixed oils, compatible with e.g. different types of injection strategies such as subcutaneous, intramuscular, intraorbital, intracapsular, intraspinal, intrasternal, intravenous etc.

In still a further embodiment, administration may be intended via dialysis.

The food and feed supplement may also be emulsified. The active therapeutic ingredient may then be mixed with excipients, which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH, buffering agents, which enhance the effectiveness of the active ingredient.

Different formats for the parenteral administration may be supplied, such as liquids or lyophilized or otherwise dried formulations. They may include diluents of various buffers (e.g., Tris-HCl, acetate, phosphate), of various pH and ionic strength, additives such as albumin or gelatine to prevent absorption to surfaces, detergents (e.g., Tween 20, Tween80, Pluronic F68, bile acid salts), solubilizing agents (e.g., glycerol, polyethyleneglycerol), anti-oxidants (e.g. ascorbic acid, sodium metabisulfite), preservatives (e.g. Thimerosal, benzyl alcohol, parabens), bulking substances or tonicity modifiers (e.g. lactose, mannitol), covalent attachment of polymers such as polyethylene glycol to the composition, complexation with metal ions, or incorporation of the material into or onto particulate preparations of polymeric compounds such as polylactic acid, polglycolic acid, hydrogels, etc., or onto liposomes, microemulsions, micelles, unilamellar or multilamellar vehicles, erythrocyte ghosts, or spheroplasts.

*A beverage.* In one embodiment, the medicament is in the form of a beverage, or a dry composition thereof, according to the invention.

The beverage comprises an effective amount of glutamin, glutamin derivates or metabolites, glutamin analogues, or a water-soluble innocuous salt thereof selected from AKG, mono- and divalent salts of AKG and ornithine-AKG, or mixtures thereof, together with a nutritionally acceptable water-soluble carrier, such as minerals, vitamins, carbohydrates, fat and proteins. All of these components are supplied in a dried form if the beverage is provided in a dry form. A beverage provided ready for consumption further comprises water. The final beverage solution may also have a controlled tonicity and acidity, e.g. as a buffered solution according to the general suggestions in the paragraph above.

The pH is preferably in the range of about 2-5, and in particularly about 2-4, to prevent bacterial and fungal growth. A sterilised beverage may also be used, with a pH of about 6-8.

The beverage may be supplied alone or in combination with one or more therapeutically effective composition(s).

*A dialysis solution. Use of a composition comprising AKG and, optionally, glucose.* According to the invention, a use of a composition comprising AKG and, optionally, glucose is includes.

One use is for the manufacture of a medicament for the prevention, alleviation or treatment of decreased muscle performance in a vertebrate, including a human bering.

One further use is for the manufacture of a medicament for modulation of muscle performance of a vertebrate, including a mammal, such as a human being, and a bird.

In a further embodiment of the above the medicament is a pharmaceutical composition. This pharmaceutical composition may be together with a pharmaceutically acceptable carrier and/or additives, such as diluents, preservatives, solubilizers, emulsifiers, adjuvants and/or carriers useful in the uses disclosed in the present invention.

Further, as used herein "pharmaceutically acceptable carriers" are well known to those skilled in the art and may include, but are not limited to, 0.01-0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Even further embodiment includes uses wherein the composition is an oral composition.

Still a further embodiment includes uses wherein the composition is a parenteral composition.

Still a further embodiment includes the use of AKG and, optionally, glucose in a therapeutic effective amount. Examples of therapeutic amounts for oral administration, parenteral administration, such as injection, and dialysis are given in the paragraph *Dose of the administered composition or pharmaceutical composition.*

A manufactured AKG composition, which may be a pharmaceutical composition or a food or feed supplement, may, optionally, comprise a carrier and/or an amount of a second or further active ingredients affecting muscle endurance.

Above mentioned effect may contribute to the increased muscle performance.

*Dose of the administered composition or pharmaceutical composition.* The AKG medicament may be for administration in different doses depending on the administration route used.

In one embodiment of the above mentioned medicaments, the AKG medicament is for administration in an amount of 5-40 mg/kg bodyweight, i.e. 5, 10, 15, 20, 25, 30, 35, 40 mg/kg bodyweight. This amount of AKG is suitable when the medicament is to be administered orally.

In a further embodiment, the AKG medicament is to be administered in an amount of 0.1-0.4 mg/kg bodyweight, i.e. 0.1, 0.2, 0.3, or 0.4 mg/kg bodyweight. This amount of AKG is suitable when AKG is to be administered parenterally.

In still a further embodiment, AKG is to be administered in an amount of 1 - 1000 mg/kg bodyweight.

In further embodiments, the optional glucose is to be administered in an amount of 0.1-5 mM, i.e. 0.1, 0.5, 1, 1.5, 2, 2.5, 3, 3.5, 4, 4.5, 5 mM.

In one embodiment, the medicament comprises AKG in an amount of 0.1-0.4 mg/kg bodyweight and glucose in an amount of 0.1-5 mM. Said medicament is suitable for parenteral administration.

In a further embodiment, the medicament comprises AKG in an amount of 5-40 mg/kg bodyweight and glucose in an amount of 0.1-5 mM. Said medicament is suitable for oral administration.

In still a further embodiment, the medicament comprises AKG in an amount of 1-1000 mg/kg bodyweight and glucose in an amount of 0.1-5 mM. Said medicament is suitable for dialysis.

*Administration targets.* As revealed above, the present invention relates to medicaments and compositions for use in treating, alleviating or preventing any condition associated with decreased muscle performance in a vertebrate, such as a mammal, including human being, or bird. Conditions that are associated with decreased muscle performance in human beings are renal patients, such as haemodialysis patients, peritoneal patients, pre-dialysis patients, and transplanted patients; osteoporosis patients; elderly, such as 70 years and above, injured/operated/bed-bound infant, such as an early premature, SGA (small for gestational age); athletes, such as cyclists, weight lifters; space flight personnel, such as astronauts in space and/or post space.

*In birds.* As can be readily appreciated by one of ordinary skill in the art, the medicaments and pharmaceutical compositions of the present invention are particularly suited for administration to any vertebrate, such as a bird or mammal in the need thereof, e.g. a human being. A vertebrate includes but is not limited to, a turkey, hen or chicken and other broilers and free going animals, or a mammal, including but not limited to, domestic animals, such as feline or canine subjects, farm animals, such as, but not limited to, bovine, equine, caprine, ovine, and porcine subjects, wild animals, whether in the wild or in a zoological garden, research animals, such as mice, rats, rabbits, goats, sheep, pigs, dogs, cats, etc.

### EXAMPLES

### Example 1

It is well known that muscle endurance can be affected by the concentration of Na-channels and Na,K-pumps. Indeed, changes in channel or pump concentration and/or gating/pumping properties with age, disease or drug administration can affect the contractile performance of muscles quite dramatically and often reversibly. The figure below shows the effects of T3 administration inducing a rapid upregulation of Na-channels preceded by an upregulation of Na,K-pumps - note the rapid rate of fatigue associated with a greater Na-influx and inadequate Na,K-pumping capacity in these muscles (Harrison & Clausen, 1998).

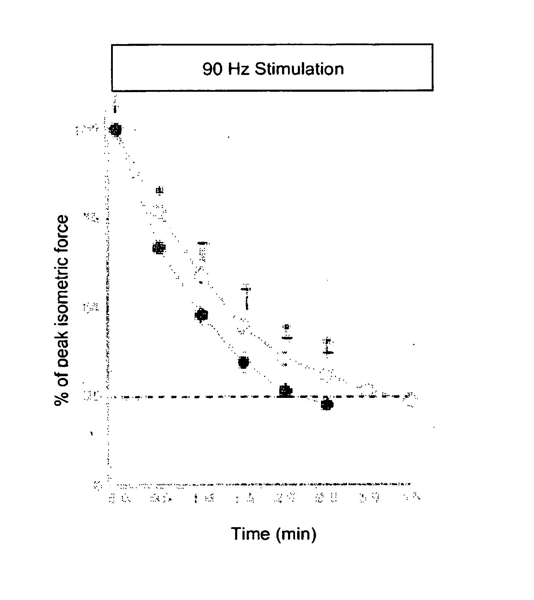

The Example is illustrated in fig. 1, presenting the time course of decline in isometric force in soleus muscles from control (O) and 48-h 3,5,3'-triiodothyronine (T3)-treated (●) 4-wk-old rats during continuous electrical stimulation at 90 Hz, applied until ∼80% of maximum force was lost. T3 was administered subcutaneously (day 1: 0.4 µg/g body wt/day; day 2: 0.2 µg /g body wt/day) while controls were sham injected with an equal volume of the solvent. Endurance, defined as the time needed for peak isometric force to decline by 75%, was measured by reading the intercept of the dashed line with the curve showing force decline off the x-axis. Force decline curves for soleus muscles from control and 48-h T3-treated rats can be fitted by single-exponential equations: y = exp(0.42x + 4.51), r = 0.98, and y = exp(0.60x + 4.52), r = 0.99, respectively. Each point represents mean ± SE (vertical lines) of observations on 8-18 muscles. * P < 0.001; P < 0.01; P < 0.05 compared with mean of controls (unpaired t-test).

### Animal maintenance

Animals were kept in a thermostatically controlled chamber, containing the equipment to measure contractile force in isolated rat skeletal muscle, i.e. a computer, analogue-digital transducer, amplifier, differential amplifier, stimulator, 5% oxygen and 5% carbon dioxide.

Thermostatically controlled chamber, maintained at 30°C, contained a glass diffuser for efficient oxygenation of buffer, a mounting/stimulation block with 2 pins to fix portion of fibula in place and 2 silver stimulating electrodes.

### Results

### Changes in muscle performance

The above measurements were recorded and results are given in fig. 1. The results show that AKG administration to uremic rats gives a better endurance profile for the slow-twitch soleus muscle compared with an identical muscle from a control sham operated rat being administered a placebo (Krebs Ringer) (AKG EFFECT).

Indeed, a soleus muscle from an uremic rat administered AKG and incubated in Krebs Ringer performs similarly to a soleus muscle from a control sham operated rat administered a placebo and incubated under the same conditions.

In control rats, AKG administration compared with a placebo improves the endurance of soleus muscles incubated in a Krebs Ringer (fig. 1) (AKG EFFECT).

AKG administration to uremic rats (Krebs Ringer) gives a better endurance profile for the slow-twitch soleus muscle compared with an identical muscle from an AKG administered uremic rat (Uremic buffer) (BUFFER EFFECT).

AKG administration to uremic rats (Krebs Ringer) gives a better endurance profile for the fast-twitch extensor digitorum longus muscle (EDL) compared to an identical muscle from a uremic rat administered AKG but incubated in Uremic buffer (BUFFER EFFECT).

EDL muscles seem to be relatively unaffected by a period of incubation in a Uremic buffer (n=2).

### INTERPRETATION

AKG administration appears to have a beneficial effect on muscle performance in terms of the endurance during the sustained contraction.

The buffer composition in which the muscle is incubated has an effect on muscle function that is independent of AKG.

### Example 2

In uremic patients suffering from chronic renal failure there is excessive muscle tiredness which affects not only their professional but also recreational life. Furthermore, these patients suffer from muscle atrophy of predominantly type II (fast-twitch) fibres. It is known that anemia is a problem in these patients but this cannot account for the muscle fibre loss or muscle weakness, likewise data suggest that local metabolic factors are not affected (ATP and creatine phosphate concentrations normal at rest in uremic rats) and that there is no mitochondrial abnormality.

One difference that does exist, however, is that of insulin resistance in chronic renal failure patients. This condition is partially improved by haemodialysis but is independent of hyperparathyroidism which influences only insulin secretion. In such patients, basal glucose transport is normal as is the amount of transporters sensitive to insulin. It does appear that muscle blood flow is reduced in these patients, however, and this will have an impact on glucose delivery and uptake by muscle fibres.

In view of the above it seems clear that we are dealing with two separate aspects - the first is a question of energy supply since insulin resistance will reduce the glucose available for the deep muscle beds of the body. Here we might anticipate that AKG will directly supply muscle fibres with an energy component that fits into the TCA cycle prior to a key energy yielding step. This hypothesis fits well with the response we have seen for control rats fed AKG compared with control rats given a placebo. The second aspect is a question of bathing medium since a Uremic Buffer induces a loss of force (endurance) that can be reversed upon incubation in a Krebs Ringer. Indeed, incubation of uremic muscles in a Krebs Ringer can restore a great deal of the lost endurance that has been recorded; in other words, we impose a form of dialysis treatment and observe a similar recovery of muscle function to that found with post- versus pry-dialysis human subjects.

In view of the second aspect, it now seems certain from isolated muscle studies that the loss of endurance is buffer related, i.e. has something to do with the build-up of compounds ions etc. arising from renal failure. We know that K-ions can and do affect muscle membranes, as do Ca-ions, so in this instance of our research we have chosen to omit these from the composition of the Uremic Buffer. In contrast, bicarbonate, urea and phosphate concentrations were elevated compared with the Krebs Ringer. It is known that membranes are excited through the operation of the Na,K-pump and the build up of phosphate alone is inhibitory to normal Na,K-pumping thereby leading to loss of excitability and hence a faster loss of force (endurance) than that observed for isolated muscles of control rats. The build up of phosphate leads to impaired excitability of muscle fibres, affecting those most dependent on the pumps (i.e. fast-fibres) initially and resulting in fibre damage/atrophy over a period of time.

### Results

1. Insulin resistance as a result of reduced muscle blood flow will have an impact on type I & II diabetes, arterial hypertension or renal failure patients alike - here AKG can be used as a means of replenishing energy, specifically in muscle being more diffusable than glucose (transporter needed) as opposed to a ion coupled exchange system and perhaps as a means of increasing the capillary bed to large muscles.
2. AKG which has been shown to reduce phosphate levels in plasma will alleviate the phosphate effect on muscle fibre membranes (& other cells) and should delay, if not stop, the progression of uremia.
3. AKG administration to sports persons will increase their endurance performance within a period of a few days to weeks in a natural and nonaddictive way.

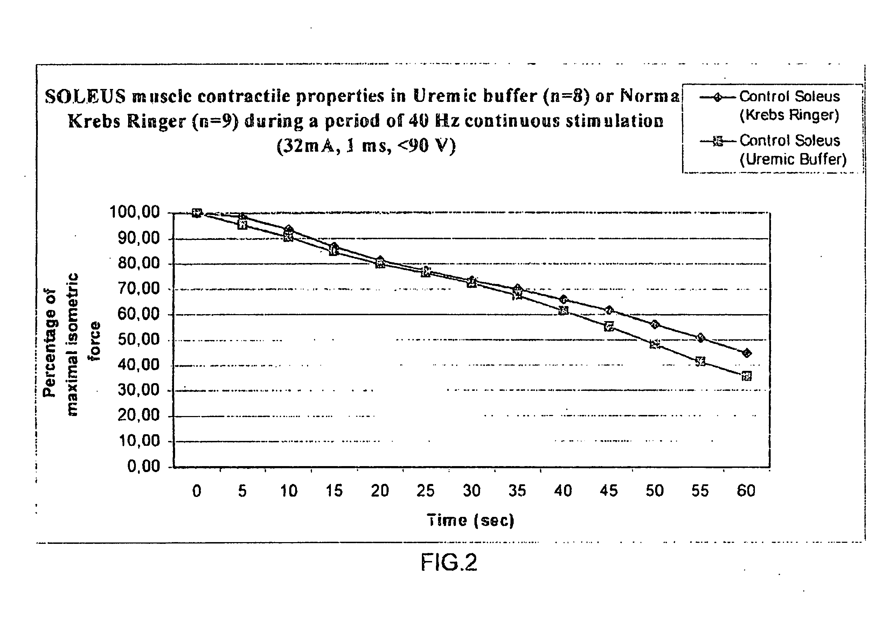

Fig. 2 presents a diagram for a soleus muscle control group (Krebs Ringer) - curve I, and for a soleus muscle control group (Uremic Buffer) - curve II, showing minimal effects of Uremic Buffer on muscle endurance in the slow-twitch soleus muscle - note that first signs of fatigue begin to show after considerable time (30-40 sec) with continuous stimulation. This finding supports the published data from patients in which the slow muscles are less affected during chronic renal failure compared with fast muscles.

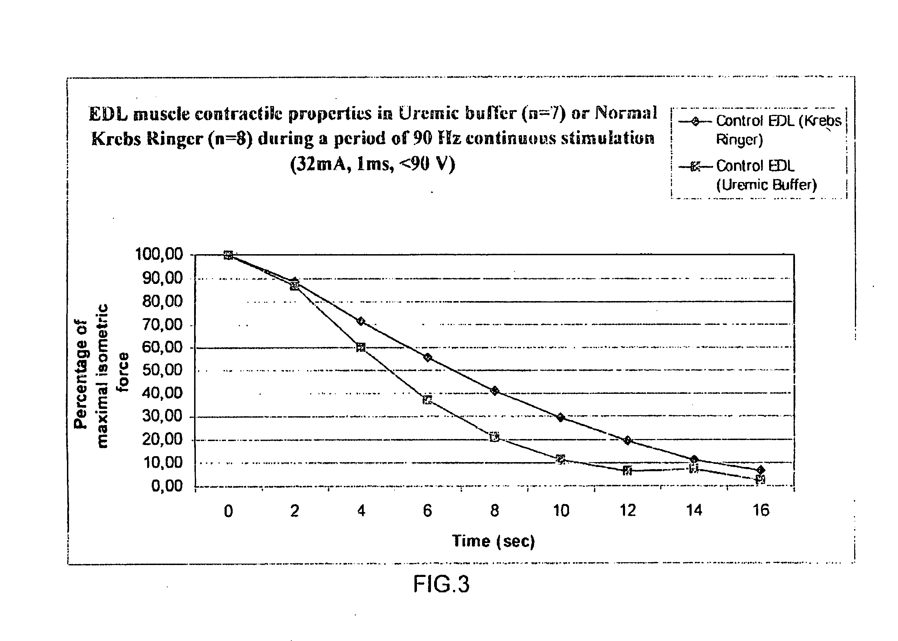

Fig. 3 presents rapid effects of Uremic Buffer on muscle endurance in the fast-twitch extensor digitorum longus muscle for a extensor digitorum longus muscle control group (Krebs Ringer) - curve I, and - for a extensor digitorum longus muscle control group (Uremic Buffer) - curve II. Note that the first signs of fatigue begin to show after only 2-4 sec of continuous stimulation.

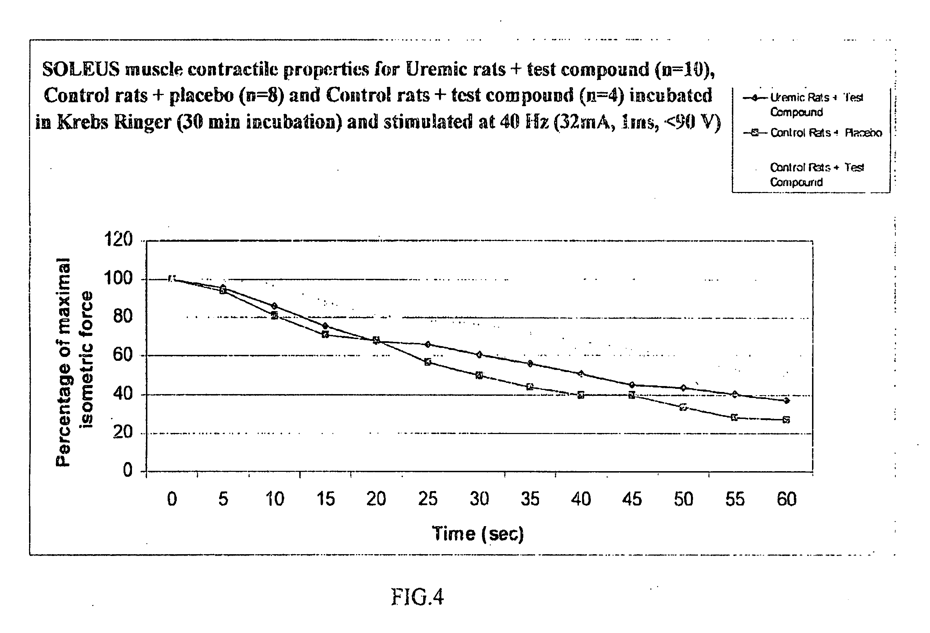

Fig. 4 presents considerable effects of AKG administration to control soleus muscles in terms of improved endurance - curve I; curve I relating to uremic rats + test component, curve II to control rats + placebo, curve III to uremic rats + test component. Note that the uremic rats administered AKG show a similar to slightly improved endurance compared with muscles from control rats given a placebo.

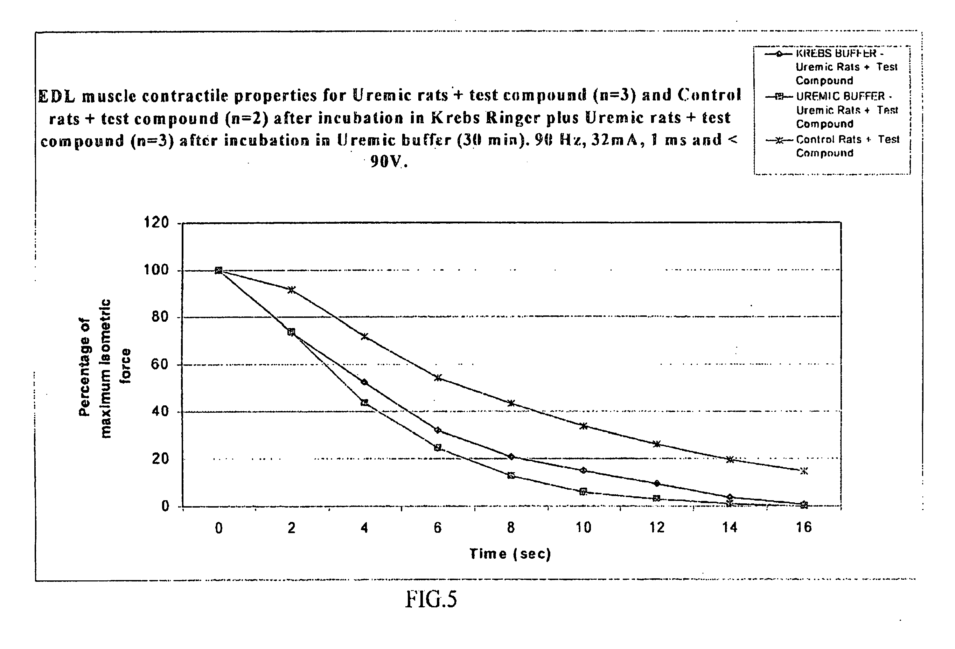

Fig. 5 presents AKG effects on endurance in extensor digitorum longus muscle for uremic rats compared with placebo treated uremic and control rats; curve I relating to uremic rats + test component (Krebs Buffer), curve II to uremic rats + test component (Uremic Buffer), curve III to control rats + test component. Note the improved performance of the AKG administered uremic rats versus the placebo administered uremic rats.

### Conclusions

1) AKG is an oral preparation that improves muscle function within a period of 21 days or less.
2) AKG given orally has a muscle and/or fibre specific effect on contractile performance which is clearly different between fast- and slow-twitch muscles.
3) AKG acts to reduce the "peripheral" fatigue that occurs when muscles contract for a sustained period of time at their normal working frequency.
4) Maximal Isometric Force (N): AKG oral treatment (21 days or less) improves the force generated in fast-twitch muscles by 28% compared with untreated controls and by 12% in slow-twitch muscles.
5) Rate of Force Generation: AKG oral treatment (21 days or less) slows the rate of force generation by some 8.5% in fast-twitch muscles and by some 130% in slow-twitch muscles.

### AKG mode of action:

AKG acts on muscle contractile performance at a number of levels; improves fatigue resistance, improves strength, and slows down the rate of force generation.
o Membrane potential: an obvious way of explaining the improved fatigue resistance is one in which AKG increases the number of active membrane-bound Na⁺,K⁺-ATPase's. Such units are responsible for maintaining the membrane resting potential and, hence, fibre excitablity. In experiments where the pump activity is increased or inhibited, the fatigue resistance is markedly improved or impaired, respectively.
o Increased muscle bulk: an increase in the size of fibres and the number of contractile units per fibre would greatly improve the isometric force of muscles. In this respect one might envisage AKG having an anabolic effect on muscle hypertrophy. Alternatively, one might suggest that AKG affects the cross-bridge cycling such that an improved force production is attained. It is known for example that a change in phosphorylation of RLC's can affect not only the force generated but also the rate of force generation in a muscle-specific fashion.
o Fibre type changes: alternatively, one might predict a change in fibre-type with AKG treatment. Indeed, the slower rise time for both muscles would be supported by an increase in the type I MHC composition of fibres or indeed by a change in Ca⁺⁺-ATPase isoforms towards those of the slow-twitch fibres. A slower Ca⁺⁺ release is for example the cause of the slower rise to peak force.

## Claims

1. Medicament comprising alpha-ketoglutarate (AKG) or a salt thereof selected from a mono- or divalent metal salt of AKG and ornithine-AKG, for use in the prevention, alleviation or treatment of decreased muscle performance in a subject.

2. The medicament according to claim 1, wherein the subject is selected from the group consisting of: a rodent, such as a mouse, rat, guinea pig or a rabbit; farm animals, such as a cow, horse, pig, piglet; a bird, such as a hen and turkey; free going farm animals or birds; a pet, such as a dog or cat; and a human being.

3. The medicament according to any of the preceding claims, wherein the medicament is a pharmaceutical composition comprising pharmaceutically acceptable carriers and/or additives in addition to the active ingredient(s) according to claim 1.

4. The medicament according to any of the preceding claims, wherein the medicament is an oral composition, parenteral composition or a composition for administration via dialysis.

5. The medicament according to any of the preceding claims, wherein the medicament is to be administered orally at 5-40 mg/kg bodyweight/day.

6. The medicament according to any of the preceding claims, wherein the medicament is to be administered parenterally at 0.1-0.4 mg/kg bodyweight/day.

7. The medicament according to claim 5, wherein the medicament is to be administered via dialysis at 1-1000 mg/kg bodyweight/day.

8. The medicament according to any of the preceding claims, wherein the medicament further comprises glucose in an amount of 0.1-5 mM.

9. The medicament according to any of the preceding claims, wherein the medicament is for use in the treatment of muscle tiredness, such as in uremic patients, such as in uremic patients suffering from chronic renal failure.

10. The medicament according to any of the preceding claims, wherein the medicament is for use in the treatment of muscle atrophy, such as in uremic patients, such as in uremic patients suffering from chronic renal failure.

11. The medicament according to any of the preceding claims, wherein the medicament is for use in the treatment of a subject selected from: a renal patient, such as haemodialysis patient, peritoneal patient, pre-dialysis patient, and transplanted patient; osteoporosis patient; elderly patient such as 70 years and above; injured/operated/bed-bound infant, such as an early premature infant or small for gestational age-infant; an athlete, such as a cyclist or a weight lifter; an astronaut in space or post space.

12. Use of alpha-ketoglutarate (AKG) or a salt thereof selected from a mono-or divalent metal salt of AKG and ornithine-AKG, for the manufacture of a medicament for the prevention, alleviation or treatment of decreased muscle performance in a subject.

## Patentansprüche

1. Medikament, umfassend α-Ketoglutarat (AKG) oder ein Salz davon, ausgewählt aus einem ein- oder zweiwertigen Metallsalz von AKG und Ornithin-AKG, zur Verwendung bei der Prävention, Linderung oder Behandlung von verminderter Muskelleistung bei einem Patienten.

2. Das Medikament nach Anspruch 1, wobei der Patient aus der Gruppe bestehend aus:
einem Nager, wie z.B. einer Maus, Ratte, einem Meerschweinchen oder Kaninchen; Bauernhoftieren, wie z.B. einer Kuh, einem Pferd, Schwein, Ferkel; einem Vogel, wie z.B. einer Henne und einem Truthahn; freilaufenden Bauernhoftieren oder Vögeln; einem Haustier, wie z.B. einem Hund oder einer Katze; und einem Menschen ausgewählt ist.

3. Das Medikament nach einem der vorhergehenden Ansprüche, wobei das Medikament ein Arzneimittel ist, das pharmazeutisch verträgliche Träger und/oder Additive zusätzlich zu dem Wirkstoff/den Wirkstoffen nach Anspruch 1 umfasst.

4. Das Medikament nach einem der vorhergehenden Ansprüche, wobei das Medikament eine orale Zusammensetzung, parenterale Zusammensetzung oder eine Zusammensetzung zur Verabreichung über Dialyse ist.

5. Das Medikament nach einem der vorhergehenden Ansprüche, wobei das Medikament oral mit 5-40 mg/kg Körpergewicht/Tag zu verabreichen ist.

6. Das Medikament nach einem der vorhergehenden Ansprüche, wobei das Medikament parenteral mit 0,1-0,4 mg/kg Körpergewicht/Tag zu verabreichen ist.

7. Das Medikament nach Anspruch 5, wobei das Medikament über Dialyse mit 1-1000 mg/kg Körpergewicht/Tag zu verabreichen ist.

8. Das Medikament nach einem der vorhergehenden Ansprüche, wobei das Medikament weiter Glucose in einer Menge von 0,1-5 mM umfasst.

9. Das Medikament nach einem der vorhergehenden Ansprüche, wobei das Medikament zur Verwendung bei der Behandlung von Muskelermüdung, wie z.B. bei urämischen Patienten, wie z.B. bei urämischen Patienten, die an chronischem Nierenversagen leiden, bestimmt ist.

10. Das Medikament nach einem der vorhergehenden Ansprüche, wobei das Medikament zur Verwendung bei der Behandlung von Muskelatrophie, wie z.B. bei urämischen Patienten, wie z.B. bei urämischen Patienten, die an chronischem Nierenversagen leiden, bestimmt ist.

11. Das Medikament nach einem der vorhergehenden Ansprüche, wobei das Medikament zur Verwendung bei der Behandlung eines Patienten, ausgewählt aus: einem Nierenpatienten, wie z.B. einem Hämodialyse-Patienten, einem peritonealen Patienten, einem Prädialyse-Patienten und einem transplantierten Patienten; einem Osteoporose-Patienten, einem älteren Patienten, wie z.B. einem 70-Jährigen und darüber; einem verletzten/operierten/bettlägerigen Kind, wie z.B. einem frühen Frühgeborenen oder einem im Schwangerschaftsstadium zu kleinen Kind; einem Athleten, wie z.B. einem Radsportler oder einem Gewichtheber; einem Astronauten im Weltraum oder nach der Rückkehr aus dem Weltraum, bestimmt ist.

12. Verwendung von α-Ketoglutarat (AKG) oder eines Salzes davon, ausgewählt aus einem ein- oder zweiwertigen Metallsalz von AKG und Ornithin-AKG, zur Herstellung eines Medikaments zur Prävention, Linderung oder Behandlung von verminderter Muskelleistung bei einem Patienten.

## Revendications

1. Médicament comprenant de l'alpha-cétoglutarate (AKG) ou un sel de celui-ci choisi parmi un sel de métal mono- ou divalent d'AKG et d'ornithine-AKG, pour une utilisation dans la prévention, l'atténuation ou le traitement d'une performance musculaire abaissée chez un sujet.

2. Médicament selon la revendication 1, dans lequel le sujet est choisi dans le groupe constitué : d'un rongeur, tel qu'une souris, un rat, un cochon d'inde ou un lapin ; des animaux de la ferme, tels qu'une vache, un cheval, un cochon, un porcelet ; un volatile, tel qu'une poule et une dinde ; des animaux de la ferme en liberté ou des oiseaux ; un animal domestique, tel qu'un chien ou un chat ; et un être humain.

3. Médicament selon l'une quelconque des revendications précédentes, dans lequel le médicament est une composition pharmaceutique comprenant des supports et/ou des additifs pharmaceutiquement acceptables en plus du(des) ingrédient(s) actif(s) selon la revendication 1.

4. Médicament selon l'une quelconque des revendications précédentes, dans lequel le médicament est une composition orale, une composition parentérale ou une composition destinée à l'administration via une dialyse.

5. Médicament selon l'une quelconque des revendications précédentes, dans lequel le médicament doit être administré par voie orale à 5-40 mg/kg de poids corporel/jour.

6. Médicament selon l'une quelconque des revendications précédentes, dans lequel le médicament doit être administré par voie parentérale à 0,1-0,4 mg/kg de poids corporel/jour.

7. Médicament selon la revendication 5, dans lequel le médicament doit être administré via une dialyse à 1-1 000 mg/kg de poids corporel/jour.

8. Médicament selon l'une quelconque des revendications précédentes, dans lequel le médicament comprend de plus du glucose dans une quantité de 0,1-5 mM.

9. Médicament selon l'une quelconque des revendications précédentes, dans lequel le médicament est destiné à une utilisation dans le traitement d'une fatigue musculaire, telle que chez des patients urémiques, comme des patients urémiques souffrant d'un défaut rénal chronique.

10. Médicament selon l'une quelconque des revendications précédentes, dans lequel le médicament est destiné à une utilisation dans le traitement de l'atrophie musculaire, telle que chez des patients urémiques, comme des patients urémiques souffrant d'un défaut rénal chronique.

11. Médicament selon l'une quelconque des revendications précédentes, dans lequel le médicament est destiné à une utilisation dans le traitement d'un sujet choisi parmi : un patient souffrant d'un trouble rénal, tel qu'un patient d'hémodialyse, un patient souffrant d'un trouble péritonéal, un patient en pré-dialyse et un patient transplanté ; un patient souffrant d'ostéoporose ; un patient âgé tel que de 70 ans et plus ; un enfant blessé/opéré/alité, tel qu'un enfant grand prématuré ou un enfant petit pour l'âge de gestation ; un athlète, tel qu'un cycliste ou un haltérophile, un astronaute dans l'espace ou après un voyage dans l'espace.

12. Utilisation d'alpha-cétoglutarate (AKG) ou d'un sel de celui-ci choisi parmi un sel de métal mono- ou divalent d'AKG et d'ornithine-AKG, pour la fabrication d'un médicament destiné à la prévention, l'atténuation ou le traitement d'une performance musculaire abaissée chez un sujet.
